# EUROPEAN PATENT APPLICATION

(11) **EP 4 299 057 A1**
(43) Date of publication of application: **03.01.2024**
(21) Application number: 22305965.0
(22) Date of filing: 30.06.2022
(51) Int. Cl.: A61K 9/48, A61K 47/18, A61P 1/00, A61P 3/10, A61P 5/50

(54) **SOLID COMPOSITIONS COMPRISING A PEPTIDE OR A PROTEIN AND AN ACYLATED AMINO ACID**

(71) Applicant: Adocia, 69003 Lyon (FR)
(72) Inventor: LAURENT, Nicolas, 01700 Miribel (FR); NOËL, Romain, 69100 Villeurbanne (FR); NAESSENS, Ulysse, 69250 Curis au Mont d'Or (FR)
(74) Representative: Casalonga

(57) **Abstract**

The invention relates to solid composition comprising a peptide or a protein and an acylated aminoacid AC-aa, or a salt thereof, characterized in that:
- the acyl group -AC comprises from 4 to 9 carbon atoms,
- said composition comprises at least 300 mg/g of AC-aa relative to the total weight of the composition.

## Description

The present invention concerns compositions comprising a peptide and a permeation enhancer, said permeation enhancer being an acylated aminoacid, also called AC-aa, their method of preparation and their use in medicine.

Current peptides or protein therapies rely mainly on the parenteral way of administration.

Although oral delivery is clearly a must have for the treatments, in particular in terms of comfort of administration and improved compliance to the treatment, the hurdles for obtaining an oral way of administration for peptides and proteins are numerous and very challenging.

Among these hurdles can be cited a low bioavailability, a great variability of the bioavailability, a difficult processability, a slow solubilization of the permeation enhancer and/or an absorption site which can be aggressive for the active principle.

The peptides or proteins which are marketed under oral form are mostly small cyclic non-acylated or non-pegylated peptides. By small is meant a molecular weight of less or equal to 1200 Da. There we can cite Cyclosporin, Octreotide and Desmopressin.

However when talking about peptides or proteins not falling into this class, we can only cite Rybelsus^{®} as marketed peptide product which is a long acting GLP-1 RA (semaglutide) combined with a permeation enhancer (SNAC) in order to allow the oral delivery.

Although this product is marketed it still has some drawbacks, such as a low bioavailability, a great variability of bioavailability between different administrations, a slow solubilization of the permeation enhancer and also a constraint regarding the dosage protocol (fasting at least 30 minutes after Rybelsus^{®} dosage).

Another prior art is Novo's patent application on FA-aa, for example WO2012140155A1, WO2014060447A1, WO2014060472A1, WO2014060512A1, WO2015162195A1. These applications are about acylated amino acids for oral delivery of peptides. They mainly disclose aminoacid acylated with fatty acid comprising at least 10 carbon atoms in liquid formulations, and most of these applications do not show results about FA-aa having a rather small fatty acid chains.

WO2014060512A1 which has data on "short" fatty acid teaches that the strongest effect is observed for FA-aa's with longer fatty acid chains (Example 12) and that the FA-aa with a fatty acid chain having 8 to 10 carbon atoms have a very modest, if any effect on the transport of Growth Hormones compounds (Example 20). As an example the table M from Example 20 shows that a composition comprising the active principle and a FA-aa with a fatty acid chain comprising 8 carbon atoms has almost no effect on delivery of the active principle, as the fold increase of delivery compared to a composition comprising the active principle alone is around 0.6 to 1.2. For most peptides, this increase in bioavailability would be far from leading to therapeutically efficient delivered doses of peptide. As the bioavailability with an oral administration of a peptide alone is very close to zero, a small multiple (and in the instant case it is very small, ie 0.6 to 1.2) of this bioavailability means that there is almost no peptide delivered.

The invention proposes a way to solve at least part of the above cited problems.

In particular the invention aims at:
- improving the bioavailability and/or
- having a very short window of absorption and/or
- decreasing the variability of bioavailability between administrations.

This last feature would in particular allow to use short acting APIs as a great variability of absorption for short acting APIs would lead to great variability of API concentration in the blood, and thus the risk that the patient has a too low or too large dose of API.

This is surprisingly that the applicant has found that a composition according to the invention solves at least one of the technical problems cited above.

This is also surprisingly that the applicant has found that a solid composition, in particular in an oral dosage form comprising the composition according to the invention, solves at least one of the technical problems cited above.

Moreover the applicant found an unexpected property of acylated aminoacid AC-aa, or a salt thereof, wherein the acyl group AC comprises from 4 to 9 carbon atoms, in particular 4 to 8 carbon atoms, as these compounds are protease inhibitors.

The composition according to the invention relates to a solid composition comprising a peptide or a protein and an acylated aminoacid AC-aa, or a salt thereof, wherein the acyl group AC comprises from 4 to 9 carbon atoms, in particular 4 to 8 carbon atoms.

The composition according to the invention relates also to a solid composition comprising a peptide or a protein and an acylated aminoacid AC-aa, or a salt thereof, wherein the acyl group is (C(O)R₁) with R1 being an alkyl group comprising 3 to 8 carbon atom s.

In an embodiment, the composition according to the invention relates also to a solid composition comprising a peptide or a protein and an acylated aminoacid AC-aa, or a salt thereof, wherein the acyl group is (C(O)R₁) with R1 being an alkyl group comprising 3 to 7 carbon atoms.

In an embodiment, the acyl group (C(O)-R₁) also called AC comprises from 4 to 9 carbon atoms, in particular from 4 to 8 carbon atoms, and said composition comprises at least 300 mg/g of AC-aa relative to the total weight of the composition.

In an embodiment, the composition comprises at least 400 mg/g of AC-aa relative to the total weight of the composition.

In an embodiment, the composition comprises at least 500 mg/g of AC-aa relative to the total weight of the composition.

In an embodiment, the composition comprises at least 600 mg/g of of AC-aa relative to the total weight of the composition.

In an embodiment, the composition comprises at least 700 mg/g of of AC-aa relative to the total weight of the composition.

In an embodiment, the composition comprises at least 800 mg/g of of AC-aa relative to the total weight of the composition.

In an embodiment, the composition comprises at least 900 mg/g of of AC-aa relative to the total weight of the composition.

In an embodiment, the composition comprises at most 990 mg/g of of AC-aa relative to the total weight of the composition.

In an embodiment, the composition comprises at most 980 mg/g of of AC-aa relative to the total weight of the composition.

In an embodiment, the composition comprises at most 950 mg/g of of AC-aa relative to the total weight of the composition.

In an embodiment, the composition comprises at most 900 mg/g of of AC-aa relative to the total weight of the composition.

In an embodiment, the composition comprises at most 800 mg/g of of AC-aa relative to the total weight of the composition.

The composition according to the invention relates to a solid composition comprising a peptide or a protein and an acylated aminoacid, also called AC-aa, or a salt thereof, wherein the acyl group comprises from 4 to 9 carbon atoms, in particular from 4 to 8 carbon atoms.

The composition according to the invention relates to a solid composition comprising a peptide or a protein, an acylated aminoacid, also called AC-aa, or a salt thereof, wherein the acyl group comprises from 4 to 9 carbon atoms, in particular from 4 to 8 carbon atoms, and a lubricant.

The composition according to the invention relates to a solid composition comprising a peptide or a protein, an acylated aminoacid, also called AC-aa, or a salt thereof, wherein the acyl group comprises from 4 to 9 carbon atoms, in particular from 4 to 8 carbon atoms, and a pH modifier.

The invention also relates to a unitary solid dosage comprising or consisting of the composition of the invention.

In an embodiment, the unitary solid dosage comprises a peptide or a protein and at least 300 mg/g of acylated aminoacid, also called AC-aa, or a salt thereof, wherein the acyl group AC comprises from 4 to 9 carbon atoms, in particular from 4 to 8 carbon atoms.

The invention also relates to a unitary solid dosage comprising a peptide or a protein and at least 300 mg/g of acylated aminoacid, also called AC-aa, or a salt thereof, wherein the acyl group AC comprises from 4 to 9 carbon atoms, in particular from 4 to 8 carbon atoms, said unitary solid dosage comprising at least 50 mg of AC-aa.

The invention also relates to a pharmaceutical formulation comprising a solid composition as disclosed in this specification.

The invention also relates to a solid composition for oral delivery comprising a peptide or a protein and an acylated aminoacid, also called AC-aa, or a salt thereof, wherein the acyl group comprises from 4 to 9 carbon atoms, in particular from 4 to 8 carbon atoms.

The invention also relates to a method of treatment comprising the step of orally taking a solid composition as disclosed in the specification for preventing or treating a disease.

In the instant specification AC-aa is a permeation enhancer.

According to an embodiment the composition is an oral composition.

The AC-aa are also abbreviated like Cation-Trigram or Quadrigram of the aa-number of carbon of the acyl. For example the sodium salt of N-octanoyl phenylalanine is abbreviated NaPheC8 and the sodium salt of N-hexanoyl phenylglycine is abbreviated NaPGlyC6.

By "peptides" is meant amides derived from two or more amino carboxylic acid molecules (the same or different) by formation of a covalent bond from the carbonyl carbon of one to the nitrogen atom of another with formal loss of water. The term is usually applied to structures formed from α-amino acids, but it includes those derived from any amino carboxylic acid. This definition comes from IUPAC gold book (https://goldbook.iupac.org/terms/view/P04479).

By "polypeptides" is meant peptides containing ten or more amino acid residues. Polypeptides are specific type of peptides.

In the instant specification and unless otherwise stated "peptides" and "polypeptides" have a molecular weight of less than or equal to 10 000.

By "proteins" is meant naturally occurring or synthetic polypeptides having molecular weight greater than about 10 000. This definition comes from IUPAC gold book.

The term « amino acid » comprises the amino acids encoded by the genetic code and amino acids not coded by the genetic code and synthetic amino acids.

Among natural amino acids not coded by the genetic code may be cited gamma-carboxyglutamate, hydroxyproline (Hyp), ornithine (Orn), 2,4-diaminobutyrate (Dab), 2,3-diaminoproprionate (Dap), sarcosine (Sarc) and phosphoserine.

Among synthetic amino acids may be cited alpha-aminoisobutyric acid (Aib), alpha-aminobutyric acid (Abu), tert-butyl-glycine, beta-alanine, 3-amino benzoic acid, 4-amino benzoic acid, anthranilic acid and phenyglycine (PGly).

By "acylated aminoacid AC-aa", is meant a compound obtained by acylation of an amino acid with an acid, and -aa is issued from an aminoacid and AC is issued from an acid.

In an embodiment, the aa is issued from a L-aminoacid. By L-aminoacid is meant that the enantiomeric excess is at least 50%, in particular at least 75%, more specifically at least 90%, or even more specifically more than 95%.

In an embodiment the aa is issued from a racemic mixture.

In an embodiment, the aa of AC-aa is issued from a non cationic amino acid, a non polar hydrophobic amino acid, a polar non charged amino acid or a polar acidic amino acid.

In an embodiment, the aa of AC-aa is issued from a non cationic amino acid, selected from the group consisting of Alanine (Ala), Valine (Val), Leucine (Leu), Isoleucine (Ile), Phenylalanine (Phe), N-methyl Phenylalanine (NMePhe), pipecolinic acid (Pip, Phenylglycine (PGly), Tryptophane (Trp), Methionine (Met), Proline (Pro), Sarcosine (Sarc), Serine (Ser), Threonine (Thr), Cysteine (Cys), Tyrosine (Tyr), Asparagine (Asn), Glutamine (Gln), Aspartic acid (Asp) and Glutamic acid (Glu).

In an embodiment, the aa of AC-aa is issued from a non polar hydrophobic amino acid selected from the group consisting of Alanine (Ala), Valine (Val), Leucine (Leu), Isoleucine (Ile), Phenylalanine (Phe), N-methyl Phenylalanine (NMePhe), pipecolinic acid (Pip), Phenylglycine (PGly), Tryptophane (Trp), Methionine (Met), Proline (Pro) and Sarcosine (Sarc).

In an embodiment, the aa of AC-aa is issued from a non polar hydrophobic amino acid selected from the group consisting of Phenylalanine (Phe), N-methyl Phenylalanine (NMePhe), pipecolinic acid, Phenylglycine (PGly), Tryptophane (Trp) and Proline (Pro).

In an embodiment, the aa of AC-aa is issued from a polar non-charged amino acid selected from the group consisting of Serine (Ser), Threonine (Thr), Cysteine (Cys), Tyrosine (Tyr), Asparagine (Asn), and Glutamine (Gln).

In an embodiment, the aa of AC-aa is issued from an aromatic amino acid selected from the group consisting of Phenylalanine (Phe), N-methyl Phenylalanine (NMePhe), Phenylglycine (PGly), Tryptophane (Trp) and Tyrosine (Tyr).

In an embodiment, the aa of AC-aa is issued from a polar acidic amino acid selected from the group consisting of Aspartic acid (Asp) and Glutamic acid (Glu).

In an embodiment, the aa of AC-aa is issued from an aliphatic amino acid selected from the group consisting of Alanine (Ala), Valine (Val), Leucine (Leu) and Isoleucine (Ile).

In an embodiment, the aa of AC-aa is issued from of an aminoacid in the form of the L-isomer, the D-isomer or a mixture of enantiomers.

In an embodiment, the AC group comprises from 4 to 9 carbon atoms.

In an embodiment, the AC group comprises from 4 to 8 carbon atoms.

In an embodiment, the AC group comprises from 6 to 9 carbon atoms.

In an embodiment, the AC group comprises from 6 to 8 carbon atoms.

In an embodiment, the AC group comprises 9 carbon atoms.

In an embodiment, the AC group comprises 8 carbon atoms.

In an embodiment, the AC group comprises 7 carbon atoms.

In an embodiment, the AC group comprises 6 carbon atoms.

In an embodiment, the -R₁ group of the acyl group (-C(O)-R₁) is an aliphatic chain comprising from 3 to 8 carbon atoms.

In an embodiment, the -R₁ group of the acyl group (-C(O)-R₁) is an aliphatic chain comprising from 3 to 7 carbon atoms.

In an embodiment, the -R₁ group of the acyl group (-C(O)-R₁) is an aliphatic chain comprising from 5 to 8 carbon atoms.

In an embodiment, the -R1 group of the acyl group (-C(O)-R₁) is an alkyl group comprising from 5 to 7 carbon atoms.

In an embodiment, the -R₁ group of the acyl group (-C(O)-R₁) is an aliphatic chain comprising 8 carbon atoms.

In an embodiment, the -R₁ group of the acyl group (-C(O)-R₁) is an aliphatic chain comprising 7 carbon atoms.

In an embodiment, the -R₁ group of the acyl group (-C(O)-R₁) is an aliphatic chain comprising 6 carbon atoms.

In an embodiment, the -R1 group of the acyl group (-C(O)-R₁) is an alkyl group comprising 5 carbon atoms.

In an embodiment, the alkyl group of the AC is a linear, branched and/or cyclic alkyl group.

In an embodiment, the alkyl group of the AC is a linear alkyl group.

In an embodiment, the alkyl group of the AC is a branched alkyl group.

In an embodiment, the alkyl group of the AC is a cyclic alkyl group, in particular alkyl substituted cyclic alkyle.

In an embodiment, the AC group is issued from an acid chosen from octanoic acid, hexanoic acid or butanoic acid.

In an embodiment, the AC group is issued from nonanoic acid.

In an embodiment, the AC group is issued from octanoic acid.

In an embodiment, the AC group is issued from heptanoic acid.

In an embodiment, the AC group is issued from hexanoic acid.

In an embodiment, the AC group is issued from pentanoic acid.

In an embodiment, the AC group is issued from butanoic acid.

In an embodiment the AC-aa is a salt of AC-aa wherein the cationic part is chosen from the group consisting of Na⁺ and K⁺.

In an embodiment the cationic part is Na⁺.

In an embodiment the cationic part is K⁺.

In an embodiment, AC-aa is chosen amongst the AC-aa having the following general Formula I :

R3OOC-(X)-N(R2)-C(=O)R1 Formula I

wherein :
- X is
   o -CH(R4)-(CH2)n- with n being 0, 1 or 2,
      or
   o a divalent 6 carbon aromatic ring (benzene), such benzene ring bearing R3OOC- and -N(R2)-C(=O)R1 in 1,2, 1,3 or 1,4 positions, in particular the benzene group does bear only R3OOC- and -N(R2)-C(=O)R1 substituents,
- -R1 is an alkyl group comprising from 3 to 8 carbon atoms,
- -R2 is
   o an hydrogen atom (-H) or a methyl group -(CH₃) and -R4 is an amino acide side chain or
   o R2 and R4 form together a -(CH₂)ₘ- group, with m being an integer chosen from 3 and 4 when n = 0, from 2 and 3 when n = 1, and 2 when n = 2
- R3 is -H, -Na or -K.

In an embodiment, AC-aa is chosen amongst the AC-aa wherein -aa is an alpha amino acid and has the general Formula la : 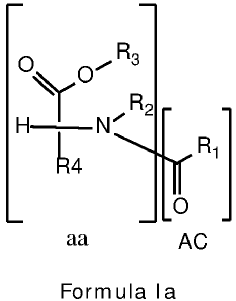 wherein :
- -R1 is an alkyl group comprising from 3 to 8, in particular from 3 to 7 carbon atoms,
- -R2 is an hydrogen atom (-H), a methyl group -(CH₃) or is covalently attached to R4 via a -(CH₂) - group,
- -R3 is -H, -Na or -K,
- -R4 is an amino acid side chain or is covalently attached to -R2 via a -(CH₂)₂₋or -(CH₂)₃- group.

In other words, R2 is covalently attached to R4 via a -(CH₂) - group and R4 is is covalently attached to -R2 via a -(CH₂)₂₋ or -(CH₂)₃- group is equivalent to R2 and R4 form together a -(CH₂)₃- or a -(CH₂)₄- group.

In an embodiment the AC-aa corresponds to the formula la wherein :
- -R1 is an alkyl group comprising from 3 to 8 carbon atoms,
- -R2 is an hydrogen atom (-H), a methyl group -(CH₃) or is covalently attached to R4 via a -(CH₂)₃₋ group,
- -R3 is -H, -Na or -K,
- -R4 is an amino acid side chain or is covalently attached to -R2 via a -(CH₂)₃₋group.

In an embodiment R2 is an hydrogen atom (-H), a methyl group -(CH₃) or is covalently attached to R4 via a -(CH₂) - group and R4 is an amino acid side chain.

In an embodiment, -R1 comprises from 3 to 8 carbon atoms.

In an embodiment, -R1 comprises from 3 to 7 carbon atoms.

In an embodiment, -R1 comprises from 5 to 8 carbon atoms.

In an embodiment, -R1 comprises from 5 to 7 carbon atoms.

In an embodiment, -R1 comprises 8 carbon atoms.

In an embodiment, -R1 comprises 7 carbon atoms.

In an embodiment, -R1 comprises 6 carbon atoms.

In an embodiment, -R1 comprises 5 carbon atoms.

In an embodiment, -R1 is a linear alkyl group.

In an embodiment, -R4 is chosen from the group consisting of -H, -CH3, - CH2CH(CH3)2, -CH(CH3)CH2CH3, -CH2-Ph (-Ph is -C6H5), -Ph, -CH2-Ph-OH (Tyrosine side chain), -CH2-Indole (-Indole is C8H6N, Tryptophan side chain), -CH2CH2SCH3, - CH2OH, -CH2SH, -CH2CONH2, -CH2CH2CONH2, -CH2COOH, -CH2CH2COOH, and their their salts.

In an embodiment the AC-aa is according to Formula I with n = 0 or to Formula la and -R4 is chosen from the group consisting of -H, -CH3, -CH2CH(CH3)2, - CH(CH3)CH2CH3, -CH2-Ph (-Ph is -C6H5), -Ph, -CH2-Ph-OH (Tyrosine side chain), - CH2-Indole (-Indole is C8H6N, Tryptophan side chain), -CH2CH2SCH3, -CH2OH, - CH2SH, -CH2CONH2, -CH2CH2CONH2, -CH2COOH, -CH2CH2COOH, and their their salts.

In an embodiment the AC-aa is according to Formula I with n = 0 or to Formula la, with R1 being -C(=O)R1 with R1 vbeing an alkyl group comprising 7 or 8 carbon atoms, and -R4 is chosen from the group consisting of -H, -CH3, -CH2CH(CH3)2, - CH(CH3)CH2CH3, -CH2-Ph (-Ph is -C6H5), -Ph, -CH2-Ph-OH (Tyrosine side chain), - CH2-Indole (-Indole is C8H6N, Tryptophan side chain), -CH2CH2SCH3, -CH2OH, - CH2SH, -CH2CONH2, -CH2CH2CONH2, -CH2COOH, -CH2CH2COOH, and their their salts.

In an embodiment, -R4 is chosen from the group consisting of -CH2-C6H5 (-C6H5 is phenyl), -Ph, -CH2-C6H4-OH (Tyrosine side chain), -CH2-Indole (-Indole is C8H6N, Tryptophan side chain).

In an embodiment the AC-aa is according to Formula I with n = 0 or to Formula la and -R4 is chosen from the group consisting of -CH2-C6H5 (-C6H5 is phenyl), -Ph, - CH2-C6H4-OH (Tyrosine side chain), -CH2-Indole (-Indole is C8H6N, Tryptophan side chain).

In an embodiment the AC-aa is according to Formula I with n = 0 or to Formula la, with R1 being -C(=O)R1 with R1 vbeing an alkyl group comprising 7 or 8 carbon atoms, and -R4 is chosen from the group consisting of-CH2-C6H5 (-C6H5 is phenyl), - Ph, -CH2-C6H4-OH (Tyrosine side chain), -CH2-Indole (-Indole is C8H6N, Tryptophan side chain).

In an embodiment, AC-aa is selected from N-butanoyl-Alanine, N-butanoyl-Valine, N-butanoyl-Leucine, N-butanoyl-Isoleucine, N-butanoyl-Phenylalanine, N-butanoyl-Phenylglycine, N-butanoyl-Tryptophane, N-butanoyl-Methionine, N-butanoyl-Proline, N-butanoyl-Sarcosine, N-butanoyl-Serine, N-butanoyl-Threonine, N-butanoyl-Cysteine, N-butanoyl-Tyrosine, N-butanoyl-Asparagine, N-butanoyl-Glutamine, N-butanoyl-Aspartic acid and N-butanoyl-Glutamic acid.

In an embodiment, AC-aa is selected from the group consisting of N-butanoyl-Alanine, N-butanoyl-Valine, N-butanoyl-Leucine, N-butanoyl-Isoleucine, N-butanoyl-Phenylalanine, N-butanoyl-Phenylglycine N-butanoyl-Tryptophane, N-butanoyl-Methionine, N-butanoyl-Proline, N-butanoyl-Sarcosine.

In an embodiment, AC-aa is selected from the group consisting of N-butanoyl-Serine, N-butanoyl-Threonine, N-butanoyl-Cysteine, N-butanoyl-Tyrosine, N-butanoyl-Asparagine and N-butanoyl-Glutamine.

In an embodiment, AC-aa is selected from the group consisting of N-butanoyl-Aspartic acid and N-butanoyl-Glutamic acid.

In an embodiment, AC-aa is selected from the group consisting of N-butanoyl-Phenylalanine, N-butanoyl-Phenylglycine, N-butanoyl-Tryptophane and N-butanoyl-Tyrosine.

In an embodiment, AC-aa is selected from the group consisting of N-hexanoyl-Alanine, N-hexanoyl-Valine, N-hexanoyl-Leucine, N-hexanoyl-Isoleucine, N-hexanoyl-Phenylalanine, N-hexanoyl-Phenylglycine, N-hexanoyl-Tryptophane, N-hexanoyl-Methionine, N-hexanoyl-Proline, N-hexanoyl-Sarcosine, N-hexanoyl-Serine, N-hexanoyl-Threonine, N-hexanoyl-Cysteine, N-hexanoyl-Tyrosine, N-hexanoyl-Asparagine, N-hexanoyl-Glutamine, N-hexanoyl-Aspartic acid and N-hexanoyl-Glutamic acid.

In an embodiment, AC-aa is selected from the group consisting of N-hexanoyl-Alanine, N-hexanoyl-Valine, N-hexanoyl-Leucine, N-hexanoyl-Isoleucine, N-hexanoyl-Phenylalanine, N-hexanoyl-Phenylglycine N-hexanoyl-Tryptophane, N-hexanoyl-Methionine, N-hexanoyl-Proline and N-hexanoyl-Sarcosine.

In an embodiment, AC-aa is selected from the group consisting of N-hexanoyl-Serine, N-hexanoyl-Threonine, N-hexanoyl-Cysteine, N-hexanoyl-Tyrosine, N-hexanoyl-Asparagine and N-hexanoyl-Glutamine.

In an embodiment, AC-aa is selected from the group consisting of N-hexanoyl-Aspartic acid and N-hexanoyl-Glutamic acid.

In an embodiment, AC-aa is selected from the group consisting of N-hexanoyl-Phenylalanine, N-hexanoyl-Phenylglycine N-hexanoyl-Tryptophane and N-hexanoyl-Tyrosine.

In an embodiment, AC-aa is selected from the group consisting N-heptanoyl-Alanine, N-heptanoyl-Valine, N-heptanoyl-Leucine, N-heptanoyl-Isoleucine, N-heptanoyl-Phenylalanine, N-heptanoyl-Phenylglycine, N-heptanoyl-Tryptophane, N-heptanoyl-Methionine, N-heptanoyl-Proline, N-heptanoyl-Sarcosine, N-heptanoyl-Serine, N-heptanoyl-Threonine, N-heptanoyl-Cysteine, N-heptanoyl-Tyrosine, N-heptanoyl-Asparagine, N-heptanoyl-Glutamine, N N-heptanoyl-Aspartic acid and N-heptanoyl-Glutamic acid.

In an embodiment, AC-aa is selected from the group consisting of N-heptanoyl-Alanine, N-heptanoyl-Valine, N-heptanoyl-Leucine, N-heptanoyl-Isoleucine, N-heptanoyl-Phenylalanine, N-heptanoyl-Phenylglycine, N-heptanoyl-Tryptophane, N-heptanoyl-Methionine N-heptanoyl-Proline and N-heptanoyl-Sarcosine.

In an embodiment, AC-aa is selected from the group consisting of N-heptanoyl-Serine, N-heptanoyl-Threonine, N-heptanoyl-Cysteine, N-heptanoyl-Tyrosine, N-heptanoyl-Asparagine and N-heptanoyl-Glutamine.

In an embodiment, AC-aa is selected from the group consisting of N-heptanoyl-Aspartic acid and N-heptanoyl-Glutamic acid.

In an embodiment, AC-aa is selected from the group consisting of N-heptanoyl-Phenylalanine, N-heptanoyl-Phenylglycine, N-heptanoyl-Tryptophane and N-heptanoyl-Tyrosine.

In an embodiment, AC-aa is selected from the group consisting N-octanoyl-Alanine, N-octanoyl-Valine, N-octanoyl-Leucine, N-octanoyl-Isoleucine, N-octanoyl-Phenylalanine, N-octanoyl-Phenylglycine, N-octanoyl-Tryptophane, N-octanoyl-Methionine, N-octanoyl-Proline, N-octanoyl-Sarcosine, N-octanoyl-Serine, N-octanoyl-Threonine, N-octanoyl-Cysteine, N-octanoyl-Tyrosine, N-octanoyl-Asparagine, N-octanoyl-Glutamine, N-octanoyl-Aspartic acid and N-octanoyl-Glutamic acid.

In an embodiment, AC-aa is selected from the group consisting of N-octanoyl-Alanine, N-octanoyl-Valine, N-octanoyl-Leucine, N-octanoyl-Isoleucine, N-octanoyl-Phenylalanine, N-octanoyl-Phenylglycine, N-octanoyl-Tryptophane, N-octanoyl-Methionine, N-octanoyl-Proline and N-octanoyl-Sarcosine.

In an embodiment, AC-aa is selected from the group consisting of N-octanoyl-Serine, N-octanoyl-Threonine, N-octanoyl-Cysteine, N-octanoyl-Tyrosine, N-octanoyl-Asparagine and N-octanoyl-Glutamine.

In an embodiment, AC-aa is selected from the group consisting of N-octanoyl-Aspartic acid and N-octanoyl-Glutamic acid.

In an embodiment, AC-aa is selected from the group consisting of N-octanoyl-Phenylalanine, N-octanoyl-Phenylglycine, N-octanoyl-Tryptophane and N-octanoyl-Tyrosine.

In an embodiment, AC-aa is selected from the group consisting N-nonanoyl-Alanine, N-nonanoyl-Valine, N-nonanoyl-Leucine, N-nonanoyl-Isoleucine, N-nonanoyl-Phenylalanine, N-nonanoyl-Phenylglycine, N-nonanoyl-Tryptophane, N-nonanoyl-Methionine, N-nonanoyl-Proline, N-nonanoyl-Sarcosine, N-nonanoyl-Serine, N-nonanoyl-Threonine, N-nonanoyl-Cysteine, N-nonanoyl-Tyrosine, N-nonanoyl-Asparagine, N-nonanoyl-Glutamine, N-nonanoyl-Aspartic acid and N-nonanoyl-Glutamic acid.

In an embodiment, AC-aa is selected from the group consisting of N-nonanoyl-Alanine, N-nonanoyl-Valine, N-nonanoyl-Leucine, N-nonanoyl-Isoleucine, N-nonanoyl-Phenylalanine, N-nonanoyl-Phenylglycine, N-nonanoyl-Tryptophane, N-nonanoyl-Methionine N-nonanoyl-Proline and N-nonanoyl-Sarcosine.

In an embodiment, AC-aa is selected from the group consisting of N-nonanoyl-Serine, N-nonanoyl-Threonine, N-nonanoyl-Cysteine, N-nonanoyl-Tyrosine, N-nonanoyl-Asparagine and N-nonanoyl-Glutamine.

In an embodiment, AC-aa is selected from the group consisting of N-nonanoyl-Aspartic acid and N-nonanoyl-Glutamic acid.

In an embodiment, AC-aa is selected from the group consisting of N-nonanoyl-Phenylalanine, N-nonanoyl-Phenylglycine, N-nonanoyl-Tryptophane and N-nonanoyl-Tyrosine.

Unless otherwise stated when "N-acyl-aa" is used in the instant specification it means either the acid form, or its salt form, in particular its sodium or potassium salt form.

In an embodiment, AC-aa has a critical micellar concentration in solution in water in biorelevant's FASSIF buffer at pH 6.5 and 25°C, also called CMC, of at least 1 mM.

The CMC is determined by surface tension measurement of aqueous solutions.

In an embodiment, the AC-aa has a CMC of at least 2.5 mM.

In an embodiment, the AC-aa has a CMC of at least 5 mM.

In an embodiment, the AC-aa has a CMC of at least 10 mM.

In an embodiment, the AC-aa has a CMC of at least 15 mM.

In an embodiment, the AC-aa has a CMC of at least 20 mM.

In an embodiment, the AC-aa has a CMC of at least 25 mM.

In an embodiment, the AC-aa has a CMC of at least 30 mM.

In an embodiment, the AC-aa has a CMC of at most 200 mM.

In an embodiment, the AC-aa has a CMC comprised in the range of 5 to 50 mM.

In an embodiment, the AC-aa has a CMC comprised in the range of 10 to 30 mM.

In an embodiment, the composition comprises at least 300 mg/g of AC-aa relative to the total weight of the composition.

In an embodiment, the composition comprises at least 400 mg/g of AC-aa relative to the total weight of the composition.

In an embodiment, the composition comprises at least 500 mg/g of AC-aa relative to the total weight of the composition.

In an embodiment, the composition comprises at least 600 mg/g of AC-aa relative to the total weight of the composition.

In an embodiment, the composition comprises at least 700 mg/g of AC-aa relative to the total weight of the composition.

In an embodiment, the composition comprises at least 800 mg/g of AC-aa relative to the total weight of the composition.

In an embodiment, the composition comprises at least 900 mg/g of AC-aa relative to the total weight of the composition.

In an embodiment, the composition comprises at most 980 mg/g of AC-aa relative to the total weight of the composition.

In an embodiment, the composition comprises at most 950 mg/g of AC-aa relative to the total weight of the composition.

In an embodiment, the composition comprises at most 900 mg/g of AC-aa relative to the total weight of the composition.

In an embodiment, the composition comprises at most 800 mg/g of AC-aa relative to the total weight of the composition.

In an embodiment, the composition according to the invention comprises at least one further permeation enhancer in addition to AC-aa.

In an embodiment the further permeation enhancer is chosen from the group consisting of caprate, in particular sodium caprate, caprylate, in particular sodium caprylate, bile salts, salcaprozate, in particular sodium salcaprozate (SNAC), glycocholate, ursodeoxycholic acid, glycochenodeoxycholate, glycodeoxycholate and their pharmaceutically acceptable salts, and mixtures thereof.

In an embodiment, the composition comprises a bile salt.According to an embodiment the composition comprises only one bile salt.

According to an embodiment the composition comprises a mixture of bile salts.

In an embodiment the bile salt is chosen from the group consisting of glycocholate, ursodeoxycholic acid, glycochenodeoxycholate, glycodeoxycholate and their pharmaceutically acceptable salts, and mixtures thereof.

According to an embodiment the bile salt is glycocholate, in particular sodium glycocholate.

In an embodiment the further permeation enhancer is chosen from the group consisting of caprate, in particular sodium caprate, caprylate, in particular sodium caprylate, bile salts, salcaprozate, and mixtures thereof in particular sodium salcaprozate (SNAC).

In an embodiment the further permeation enhancer is chosen from the group consisting of caprate, in particular sodium caprate, caprylate, in particular sodium caprylate.

In an embodiment the further permeation enhancer is chosen from the group consisting of caprate, in particular sodium caprate.

In an embodiment, the composition comprises at least 300 mg/g of permeation enhancers relative to the total weight of the composition.

In an embodiment, the composition comprises at least 400 mg/g of permeation enhancers relative to the total weight of the composition.

In an embodiment, the composition comprises at least 500 mg/g of permeation enhancers to the total weight of the composition.

In an embodiment, the composition comprises at least 600 mg/g of permeation enhancers relative to the total weight of the composition.

In an embodiment, the composition comprises at least 700 mg/g of permeation enhancers relative to the total weight of the composition.

In an embodiment, the composition comprises at least 800 mg/g of permeation enhancers relative to the total weight of the composition.

In an embodiment, the composition comprises at least 900 mg/g of permeation enhancers relative to the total weight of the composition.

In an embodiment, the composition comprises at most 990 mg/g of permeation enhancers relative to the total weight of the composition.

In an embodiment, the composition comprises at most 980 mg/g of permeation enhancers relative to the total weight of the composition.

In an embodiment, the composition comprises at most 950 mg/g of permeation enhancers relative to the total weight of the composition.

In an embodiment, the composition comprises at most 900 mg/g of permeation enhancers relative to the total weight of the composition.

In an embodiment, the composition comprises at most 800 mg/g of permeation enhancers relative to the total weight of the composition.

In an embodiment, the composition comprises a protease inhibitor different from AC-aa.

In an embodiment, the composition comprises a protease inhibitor chosen from the group consisting of serine protease inhibitor, Metalloproteases inhibitors, and mixtures thereof.

In an embodiment, the composition comprises a serine protease inhibitor.

In an embodiment, the composition comprises a serine protease inhibitor chosen from the group consisting of proteines, peptides, serpins, chemicals and mixtures thereof as listed in the following paragraphs.

In an embodiment, the composition comprises a serine protease inhibitor which is a proteine or a peptide chosen from the group consisting of Aprotinin, Bacitracin, Lima bean trypsin inhibitor, Ovomucoid, Soybean trypsin inhibitor (SBTI), KTI (Kunitz Trypsine Inhibitor), BBI (Bowman-Birk Inhibitor), and mixtures thereof.

In an embodiment, the composition comprises a serine protease inhibitor which is a serpin chosen from the group consisting of Alpha-1-antitrypsin, Alpha 1-antichymotrypsin, alpha 2-macroglobulin, Antithrombin, Centerin, Kallistatin, Neuroserpin, Pancpin, Plasminogen activator inhibitor-2, Protein C inhibitor, Secretory leucocyte protease inhibitor, Squamous cell carcinoma antigen-1, Squamous cell carcinoma antigen-2, Ulinastatin, Vaspin, and mixtures thereof.

In an embodiment, the composition comprises a serine protease inhibitor which is a chemical chosen form the group consisting of AEBSF-hydrochloride (4-(2-aminoethyl)benzenesulfonyl fluoride), Aminobenzamidine dihydrochloride, epsilon-aminocaproic acid, APMSF-hydrochloride, benzamidine-hydrochloride, camostat mesylate, chymostatin, FK448, leupeptin, PEFABLOC SC, PMSF (Phenylmethylsulfonyl fluoride), TLCK (Nα-Tosyl-Lys-chloromethylketone), TPCK (6-(1-tosylamido-2-phenyl) ethyl chloromethyl ketone), and mixtures thereof.

In an embodiment, the composition comprises a serine protease inhibitor chosen from the group consisting of SBTI, KTI, BBI, aprotinin, ovomucoid, and mixtures thereof.

In an embodiment, the composition comprises a serine protease inhibitor chosen from the group consisting of SBTI, KTI, BBI and mixtures thereof, particularly SBTI.

According to an embodiment the composition comprises only one serine protease inhibitor.

According to an embodiment, the composition comprises SBTI.

According to an embodiment, the composition comprises KTI.

According to an embodiment, the composition comprises BBI.

According to an embodiment the composition comprises a mixture of serine protease inhibitor.

According to an embodiment the composition comprises a mixture of serine protease inhibitor chosen from the group consisting of SBTI and aprotinin, KTI and aprotinin, BBI and aprotinin, ovomucoid and aprotinin, SBTI and ovomucoid, KTI and ovomucoid, BBI and ovomucoid.

In an embodiment, the composition comprises a metalloprotease inhibitor.

In an embodiment the composition comprises as metalloproteases inhibitor CPB (Carboxypeptidase) Inhibitor from potato tuber.

According to an embodiment the composition comprises only one metalloprotease inhibitor.

According to an embodiment the composition comprises a mixture of metalloprotease inhibitors.

According to an embodiment the composition comprises a mixture of serine protease inhibitor and metalloprotease inhibitor.

According to an embodiment the composition comprises the mixture of serine protease inhibitor and metalloprotease inhibitor is chosen from the group consisting SBTI and CPB inhibitor, BBI and CPB inhibitor, KTI and CPB inhibitor aprotinin and CPB inhibitor, ovomucoid and CPB inhibitor.

According to an embodiment the composition comprises a mixture of serine protease inhibitor and metalloprotease inhibitor.

According to an embodiment the composition comprises the mixture of serine protease inhibitor and metalloprotease inhibitor is chosen from the group consisting of SBTI and sodium glycocholate, aprotinin and sodium glycocholate, ovomucoid and sodium glycocholate.

According to an embodiment the composition comprises from 10 to 500 mg of protease inhibitor.

According to an embodiment the composition comprises from 10 to 200 mg of protease inhibitor.

According to an embodiment the composition comprises from 10 to 150 mg of protease inhibitor.

According to an embodiment the composition comprises from 10 to 100 mg of protease inhibitor.

According to an embodiment the composition comprises from 20 to 600 mg/g of protease inhibitor.

According to an embodiment the composition comprises from 20 to 400 mg/g of protease inhibitor.

According to an embodiment the composition comprises from 20 to 250 mg of protease inhibitor.

According to an embodiment the composition comprises from 20 to 150 mg of protease inhibitor.

In an embodiment the composition comprises SBTI, KTI, BBI and their mixtures, and in particular SBTI, from 100 to 400 mg/g.

In an embodiment the composition comprises SBTI, KTI, BBI and their mixtures, and in particular SBTI, from 120 to 300 mg/g.

In an embodiment the composition comprises SBTI, KTI, BBI and their mixtures, and in particular SBTI, from 140 to 250 mg/g.

In an embodiment the composition comprises SBTI, KTI, BBI and their mixtures, and in particular SBTI, from 150 to 200 mg/g.

In an embodiment, the composition comprises a chelator of divalent cations.

In an embodiment, this chelator is a physiologically acceptable compound having a high affinity for at least one of calcium, magnesium, and manganese ions.

In another embodiment, the chelator is selected from the group consisting of ethylenediamine tetracetic acid (EDTA) or a salt thereof (for example disodium EDTA and calcium disodium EDTA); EGTA (ethylene glycol tetraacetic acid) or a salt thereof; diethylene triamine pentaacetic acid (DTPA) or a salt thereof; BAPTA (I,2-bis(o-aminophenoxy)ethane-N,N,N',N'-tetraacetic acid) or a salt thereof; and mixtures thereof.

In another embodiment, only one of the above-listed chelators is present in the composition.

In another embodiment, the chelator is EDTA.

In an embodiment the composition comprises 1 to 50 % w/w of chelator of divalent cations, in particular EDTA.

In an embodiment the composition comprises 2 to 30 % w/w of chelator of divalent cations, in particular EDTA.

In an embodiment the composition comprises 5 to 25 % w/w of chelator of divalent cations, in particular EDTA.

In an embodiment, the peptide or protein is a therapeutic peptide or protein.

In an embodiment, the peptide or protein is long acting.

By « long acting » is meant having a half life in plasma of at least 1 day.

In an embodiment long acting peptide or protein have a half life in plasma of at least 2 days.

In an embodiment long acting peptide or protein have a half life in plasma of at least 3 days.

In an embodiment long acting peptide or protein have a half life in plasma of at least 5 days.

In an embodiment long acting peptide or protein have a half life in plasma of at least 7 days.

In an embodiment long acting peptide or protein have a half life in plasma of at least 10 days.

In an embodiment long acting peptide or protein have a half life in plasma of at least 15 days.

In an embodiment, the peptide or protein is short acting.

By « short acting » is meant having a half life in plasma of less than 1 day.

In an embodiment short acting peptide or protein have a half life in plasma of at most 18 hours.

In an embodiment short acting peptide or protein have a half life in plasma of at most 12 hours.

In an embodiment short acting peptide or protein have a half life in plasma of at most 6 hours.

In an embodiment short acting peptide or protein have a half life in plasma of at most 4 hours.

In an embodiment short acting peptide or protein have a half life in plasma of at most 2 hours.

In an embodiment, the peptide or protein has a solubility in water at 25°C or more of at least 10 mg/ml.

In an embodiment, the peptide or protein has a solubility in water at 25°C or more of at least 20 mg/ml.

In an embodiment, the peptide or protein has a solubility in water at 25°C or more of at least 30 mg/ml.

In an embodiment, the peptide or protein has a solubility in water at 25°C or more of at least 40 mg/ml.

In an embodiment, the peptide or protein has a solubility in water at 25°C or more of at least 50 mg/ml.

In an embodiment, the peptide or protein has a solubility in water at 25°C or more of at most 40 mg/ml.

In an embodiment, the peptide or protein has a solubility in water at 25°C or more of at most 30 mg/ml.

In an embodiment, the peptide or protein has a solubility in water at 25°C or more of at most 20 mg/ml.

In an embodiment, the peptide or protein has a solubility in water at 25°C or more of at most 10 mg/ml.

In an embodiment, the peptide or protein has a molecular weight of at least 1500 Da.

In an embodiment, the peptide or protein has a molecular weight of at least 2000 Da.

In an embodiment, the peptide or protein has a molecular weight of at least 2500 Da.

In an embodiment, the peptide or protein has a molecular weight of at least 3000 Da.

In an embodiment, the peptide or protein has a molecular weight of at least 3500 Da.

In an embodiment, the peptide or protein has a molecular weight of at least 4000 Da.

In an embodiment, the peptide or protein has a molecular weight of at most 20000 Da.

In an embodiment, the peptide or protein has a molecular weight of at most 15000 Da.

In an embodiment, the peptide or protein has a molecular weight of at most 10000 Da.

In an embodiment, the peptide or protein has a molecular weight of at most 5000 Da.

In an embodiment, the composition comprises from 0.25 to 20 wt% of peptide or protein.

In an embodiment, the composition comprises from 0.5 to 20 wt% of peptide or protein.

In an embodiment, the composition comprises from 1 to 20 wt% of peptide or protein.

In an embodiment, the composition comprises from 2 to 20 wt% of peptide or protein.

In an embodiment, the composition comprises from 5 to 20 wt% of peptide or protein.

In an embodiment, the composition comprises from 1 to 15 wt% of peptide or protein.

In an embodiment, the composition comprises from 2 to 15 wt% of peptide or protein.

In an embodiment, the composition comprises from 5 to 15 wt% of peptide or protein.

In an embodiment, the peptide or protein is chosen from the group consisting of GLP-1 RA, GLP-2 RA, insulin and insulin analogs, amylin RA, GIP RA, PYY RA, dual agonists GIP/GLP-1, dual agonists GLP-1/glucagon, ParaThyroid Hormones (PTH) and PTH analogs, InterLeukines (IL) and IL analogs, Growth Hormones (GH) and GH analogs, Insulin Growth Factors (IGF) and IGF analogs, Interferons (IFN) and IFN analogs.

In an embodiment, the peptide or protein is octreotide

In an embodiment, the peptide or protein is a GLP-1 RA chosen from the group consisting of semaglutide.

In an embodiment, the peptide or protein is a GLP-1 RA chosen from the group consisting of dulaglutide.

In an embodiment, the peptide or protein is a GLP-2 RA chosen from the group consisting of teduglutide.

In an embodiment, the peptide or protein is insulin or an insulin analog. In particular the insulin analog is chosen from the group consisting of degludec and detemir.

In an embodiment, the peptide or protein is an amylin RA or an amylin analog, in particular chosen from the group consisting of pramlintide, cagrilintide.

In an embodiment, the peptide or protein is a GIP RA chosen from the group consisting of GI P RA disclosed in WO2021021877, WO16066744 and WO2018181864.

In an embodiment, the peptide or protein is a PYY RA chosen from the group consisting of PYY (1-36), PYY (3-36) and PYY RA disclosed in WO2021023817, WO19147650 and WO2016198682.

In an embodiment, the peptide or protein is a dual agonist GIP/GLP-1 chosen from the group consisting of dual agonist GI P/GLP-1 disclosed in WO2016111971, and in particular Tirzepatide.

In an embodiment, the peptide or protein is a dual agonists GLP-1/glucagon.

In an embodiment, the peptide or protein is an analog of oxyntomodulin.

In an embodiment, the peptide or protein is a ParaThyroid Hormone (PTH) or a PTH analog chosen from the group consisting of human PTH and PTH analogs.

In an embodiment, the peptide or protein is an InterLeukines (IL) or IL analog chosen from the group consisting of IL-11 and analogs.

In an embodiment, the peptide or protein is a Growth Hormone (GH) or a GH analog chosen from the group consisting of Human Growth Hormone and analogs, in particular human growth hormone bearing an acylated graft.

In an embodiment, the peptide or protein is an Insulin Growth Factor (IGF) or an IGF analog chosen from the group consisting of IGF-1.

In an embodiment, the peptide or protein is an Interferon (IFN) or an IFN analog chosen from the group consisting of INF beta-1a, INF beta-1b and INF gamma.

In an embodiment, the peptide or protein is a peptide or protein comprising modifications in the form of a covalent modification such as a side chain attached to one or more amino acids of the hydrophylic peptide or protein.

In an embodiment, the peptide or protein is a peptide or protein comprising modifications in the form of an attachment of amides, carbohydrates, alkyl groups, acyl groups, esters, PEGylations and the like.

In an embodiment, the peptide or protein is a peptide or protein comprising modifications in the form of an attachment at least one acyl group, said acyl group comprising at least 10 carbon atoms.

In an embodiment, the acyl moiety is -OEG-OEG-gamma-L-Glu-octadecanedioyl, wherein OEG is -COCH₂O(CH₂)₂O(CH₂)₂NH-

In an embodiment, the peptide or protein is a peptide or protein comprising modifications in the form of an attachment at least one PEGylation.

In an embodiment the composition does not comprise Growth Hormone. In a specific embodiment, the composition does not comprise growth hormones such as disclosed in WO2014060512.

In an embodiment the composition does not comprise human Growth Hormone.

In an embodiment the composition does not comprise insulin.

In an embodiment the composition does not comprise long-acting insulin. In a specific embodiment the composition does not comprise a long-acting insulin, in particular such as disclosed in WO2005012347, WO2009063072 and WO9507931.

In another specific embodiment, the composition does not comprise peptide or protein, in particular insulins, such as disclosed in WO2012140155 and WO2014060447.

In an embodiment the composition does not comprise insulin comprising an acylated graft.

In an embodiment, the weight ratio AC-aa / peptide or protein is going from 1:1 to 200:1.

In an embodiment, the weight ratio AC-aa / peptide or protein is going from 1:1 to 100:1.

In an embodiment, the weight ratio AC-aa / peptide or protein is going from 1:1 to 50:1.

In an embodiment, the weight ratio AC-aa / peptide or protein is going from 2:1 to 40:1.

In an embodiment, the weight ratio AC-aa / peptide or protein is going from 4:1 to 30:1.

In an embodiment, the weight ratio AC-aa / peptide or protein is going from 6:1 to 20:1.

In an embodiment, the composition comprises less than 10 % w/w of water.

In an embodiment, the composition comprises less than 5 % w/w of water.

In an embodiment, the composition comprises less than 2 % w/w of water.

In an embodiment, the composition comprises less than 1 % w/w of water.

In an embodiment, the solid composition comprises a mixture of particles comprising the AC-aa and particles comprising the peptide or protein. In particular the AC-aa and the peptide or protein are present in the composition in the ratio and percentages as disclosed above.

In an embodiment, the solid composition consists of a mixture of particles comprising AC-aa and of particles comprising the peptide or protein. In particular the AC-aa and the peptide or protein are present in the composition in the ratio and percentages as disclosed above.

In an embodiment, the solid composition comprises particles wherein the AC-aa and the peptide or protein are mixed. In particular in the ratio and percentages as disclosed above.

In an embodiment, the solid composition consists of particles wherein the AC-aa and the peptide or protein are mixed. In particular in the ratio and percentages as disclosed above.

In an embodiment, the solid composition consists of particles wherein the AC-aa and the peptide or protein are mixed. In particular in the ratio and percentages as disclosed above.

The particles comprising at the same time peptide or protein and AC-aa may be obtained via lyophilization, freeze drying, spray drying, wet granulation or dry granulation.

In an embodiment, the particles are chosen in a group consisting of microgranules (5-0.5mm, 0.5 mm excluded), microparticules (0.5mm-1µm, 1 µm excluded) or nanoparticles (1000-1nm) and mixtures thereof.

In an embodiment, the particles are microgranules (number-averaged mean diameter 5.0-0.5mm), for instance measured by laser granulometry.

In an embodiment, the particles are microparticules (number-averaged mean diameter 0.5mm-1µm), for instance measured by light obscuration.

In an embodiment, the particles are nanoparticles (number-averaged mean diameter 1000-1nm), for instance measured by electron microscopy.

Among manufacturing methods to reach these specific sizes the following methods can be cited wet granulation, dry granulation, spray-drying, milling, nanoprecipitation.

In an embodiment these particles are free from each other.

In another embodiment, these particles are held together.

In an embodiment, the composition comprises excipients chosen from the list consisting of lubricants, surfactants, pH modifiers, disintegrants, binders, fillers, glidants, diluents, polymers for sustained or delayed release (e.g. Eudragit polymers manufactured by Evonik) and preservatives.

In an embodiment, the composition comprises only excipients chosen from the list consisting of lubricants, surfactants, pH modifiers, disintegrants, binders, fillers, glidants, diluents and preservatives.

In an embodiment, the composition comprises only excipients chosen from the list consisting of lubricants, pH modifiers.

In an embodiment, the composition comprises at most 20 % w/w of excipients, in particular of excipients such as listed above, more particularly components belonging to the lists in the 3 paragraphs above.

By "excipients" is meant components of the composition which are not permeation enhancers and protease inhibitors.

In an embodiment, the composition comprises at most 15 % w/w of excipients, in particular of excipients such as listed above.

In an embodiment, the total amount of binder, filler and glidant is at most 10 % w/w of the composition.

In an embodiment, the composition comprises at least one pH modifier.

In an embodiment the pH modifier can be chosen from the group consisting of sodium carbonate, which formula is Na₂CO₃, phosphates, citrates, citric acid, tartarate and tartaric acid.

Citrates can be monosodium citrate, disodium citrate and/or trisodium citrate. In particular it can be trisodium citrate.

Tartarate can be monosodium and/or disodium tartarate. In particular this is monosodium tartarate.

In an embodiment the pH modifier is sodium carbonate, which formula is Na₂CO₃.

In an embodiment the composition comprises at least 1 % w/w of pH modifier.

In an embodiment the composition comprises at least 2 % w/w of pH modifier.

In an embodiment the composition comprises at least 5 % w/w of pH modifier.

In an embodiment the composition comprises at most 15 % w/w of pH modifier.

In an embodiment the composition comprises at most 10 % w/w of pH modifier.

In an embodiment the composition comprises at most 8 % w/w of pH modifier.

In an embodiment the composition comprises at least one lubricant.

In an embodiment the lubricant is chosen from the group consisting of magnesium stearate or glyceryl dibehenate.

In an embodiment the lubricant is magnesium stearate.

In an embodiment the lubricant is glyceryl dibehenate.

In an embodiment the composition comprises more than or is equal to 0.1 % w/w of lubricant.

In an embodiment the composition comprises more than or is equal to 0.2 % w/w of lubricant.

In an embodiment the composition comprises more than or is equal to 0.5 % w/w of lubricant.

In an embodiment the composition comprises less than 5 % w/w of lubricant.

In an embodiment the composition comprises less than 3 % w/w of lubricant.

In an embodiment the composition comprises less than 2.5 % w/w of lubricant.

In an embodiment the composition comprises less than 2 % w/w of lubricant.

In an embodiment the composition comprises less than 1 % w/w of lubricant.

In an embodiment the composition comprises between 0.25 and 2.5 % w/w of lubricant.

In an embodiment, the composition is in the form of a unitary solid dosage form, such as capsules, tablets, dragees, pills, lozenges, powders, and granules.

In an embodiment, the composition is in the form of a unitary solid dosage form, such as capsules, tablets, dragees, pills, lozenges, powders, and granules, said unitary dosage form comprising at least 50 mg of AC-aa.

In an embodiment, the unitary solid dosage form is under the form of a capsule.

In an embodiment, the unitary solid dosage form is under the form of a hard capsule.

In an embodiment, the unitary solid dosage form is under the form of a soft capsule.

The capsules may contain powders, granules, crushed tablets that contains the peptide or a protein and one or more inert ingredients. Capsules can be formulated with delayed release characteristics.

In an embodiment the composition is encapsulated or the like to allow the composition which is swallowed to be in contact with the gastro intestinal system.

In an embodiment the encapsulation allows the composition to be delivered in the stomach.

Thus the composition may be in the form of a film coated tablets with a thin layer of water soluble material that dissolves rapidly in the stomach.

In another embodiment the encapsulation allows the composition to be delivered in the intestines.

Thus the composition may be encapsulated with an enteric coating. This enteric coating may be in the form of a polymer coating or of a polymer capsule that controls disintegration and release of the composition.

The enteric coating may be soft technology. In an embodiment it is soft capsule technnology.

The enteric coating may be hard technology. In an embodiment it is hard capsule technnology.

The term "enteric coating" means a coating that controls disintegration and release of the oral dosage form.

The site of disintegration and release of the solid dosage form may be designed depending on the pH of the targeted area, where absorption of the peptide or protein is desired, thus also includes acid resistant protective coatings.

In an embodiment, the solid oral dosage form allow a release at pH from 4.5 and above.

In an embodiment, the solid oral dosage form allow a release at pH from 4.75 and above.

In an embodiment, the solid oral dosage form allow a release at pH from 5.0 and above.

In an embodiment, the solid oral dosage form allow a release at pH from 5.25 and above.

In an embodiment, the solid oral dosage form allow a release at a pH from 5.5 and above.

In an embodiment, the solid oral dosage form allow a release at a pH from 5.75 and above.

In an embodiment, the solid oral dosage form allow a release at a pH from 6.0 and above.

The site of disintegration and release of the solid oral dosage form may be designed using delayed-release technologies, such as polymers with a controlled solubilization rate in aqueous medium, for instance Eudragit RL or RS polymers.

In an embodiment the Solid Oral Dosage Form according to the invention at pH 4.5 is considered as an Immediate-Release Solid Oral Dosage Form.

In an embodiment the Solid Oral Dosage Form according to the invention at pH 5.0 is considered as an Immediate-Release Solid Oral Dosage Form.

In an embodiment the Solid Oral Dosage Form according to the invention at pH 5.5 is considered as an Immediate-Release Solid Oral Dosage Form.

In an embodiment the Solid Oral Dosage Form according to the invention at pH 6.0 is considered as an Immediate-Release Solid Oral Dosage Form.

To be considered as an Immediate-Release Solid Oral Dosage Form means that the solid dosage oral form complies with the conditions disclosed in "Dissolution Testing and Acceptance Criteria for Immediate-Release Solid Oral Dosage Form Drug Products Containing High Solubility Drug Substances Guidance for Industry" - August 2018(Additional copies are available from: Office of Communications, Division of Drug Information Center for Drug Evaluation and Research Food and Drug Administration 10001 New Hampshire Ave., Hillandale Bldg., 4th Floor Silver Spring, MD 20993-0002 Phone: 855-543-3784 or 301-796-3400; Fax: 301-431-6353 Email: druginfo@fda.hhs.gov
http://www.fda.gov/Drugs/GuidanceComplianceRegulatoryInformation/Guidances/def ault.htm).

In an embodiment the solid dosage oral form comprises from 50 to 250 mg of SBTI, KTI, BBI and their mixtures, and in particular of SBTI.

In an embodiment the solid dosage oral form comprises from 60 to 200 mg of SBTI, KTI, BBI and their mixtures, and in particular of SBTI.

In an embodiment the solid dosage oral form comprises from 70 to 150 mg of SBTI, KTI, BBI and their mixtures, and in particular of SBTI.

In an embodiment, the composition is for use as a medicament.

In an embodiment the composition is for preventing or treating obesity, Diabetes Type 1, Diabetes Type 2, NASH, thrombocytopaenia, Short Bowel Syndrom (SBS), adult GH deficiency, GH disorders, Short stature syndrome, Turner's syndrome, Achondroplasia, Prader-Willi syndrome, Short stature in children, osteoporosis and hypoparathyroidism, Multiple sclerosis, Bone disorders.

In an embodiment the composition is for preventing or treating obesity.

In an embodiment the composition is for preventing or treating Diabetes Type 1.

In an embodiment the composition is for preventing or treating Diabetes Type 2.

In an embodiment the composition is for preventing or treating NASH.

In an embodiment the composition is for preventing or treating thrombocytopaenia.

In an embodiment the composition is for preventing or treating Short Bowel Syndrom (SBS).

In an embodiment the composition is for preventing or treating adult GH deficiency, GH disorders, Short stature syndrome, Turner's syndrome, Achondroplasia, Prader-Willi syndrome or Short stature in children.

In an embodiment the composition is for preventing or treating osteoporosis.

In an embodiment the composition is for preventing or treating hypoparathyroidism.

In an embodiment the composition is for preventing or treating Multiple sclerosis.

In an embodiment the composition is for preventing or treating Bone disorders.

According to an embodiment, the composition comprises the AC-aa with an AC group comprising 8 carbon atoms and a peptide or a protein.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr) and a peptide or a protein.

According to an embodiment, the composition comprises the AC-aa with an AC group comprising 8 carbon atoms, a peptide or a protein, and a lubricant. According to an embodiment, the composition comprises the AC-aa with an AC group comprising 8 carbon atoms, a peptide or a protein, a protease inhibitor, and a lubricant.

According to an embodiment, the composition comprises the AC-aa with an AC group comprising 8 carbon atoms, a peptide or a protein, and a lubricant which is chosen from the group consisting of magnesium stearate or glyceryl dibehenate.

According to an embodiment, the composition comprises the AC-aa with an AC group comprising 8 carbon atoms, a peptide or a protein and a further permeation enhancer.

According to an embodiment, the composition comprises the AC-aa with an AC group comprising 8 carbon atoms, a peptide or a protein and a further permeation enhancer which is chosen from the group consisting of caprate, in particular sodium caprate, caprylate, in particular sodium caprylate, bile salts, salcaprozate, in particular sodium salcaprozate (SNAC).

According to an embodiment, the composition comprises the AC-aa with an AC group comprising 8 carbon atoms, a peptide or a protein and a pH modifier.

According to an embodiment, the composition comprises the AC-aa with an AC group comprising 8 carbon atoms, a peptide or a protein and a pH modifier which is chosen from the group consisting of sodium carbonate, which formula is Na₂CO₃, phosphates, citrates, citric acid, tartarate and tartaric acid.

According to an embodiment, the composition comprises the AC-aa with an AC group comprising 8 carbon atoms, a peptide or a protein and a protease inhibitor..

According to an embodiment, the composition comprises the AC-aa with an AC group comprising 8 carbon atoms, a peptide or a protein, a pH modifier and a protease inhibitor.

According to an embodiment, the composition comprises the AC-aa with an AC group comprising 8 carbon atoms, a peptide or a protein, a further permeation enhancer and a protease inhibitor.

According to an embodiment, the composition comprises the AC-aa with an AC group comprising 8 carbon atoms, a peptide or a protein, a lubricant and a protease inhibitor.

According to an embodiment, the composition comprises the AC-aa with an AC group comprising 8 carbon atoms, a peptide or a protein, a pH modifier, a further permeation enhancer and a protease inhibitor.

According to an embodiment, the composition comprises N-octanoyl-Phenylalanine, a peptide or a protein, and a lubricant.

According to an embodiment, the composition comprises N-octanoyl-Phenylalanine, a peptide or a protein, and a lubricant which is chosen from the group consisting of magnesium stearate or glyceryl dibehenate.

According to an embodiment, the composition comprises N-octanoyl-Phenylalanine, a peptide or a protein and a further permeation enhancer.

According to an embodiment, the composition comprises N-octanoyl-Phenylalanine, a peptide or a protein and a further permeation enhancer which is chosen from the group consisting of caprate, in particular sodium caprate, caprylate, in particular sodium caprylate, bile salts, salcaprozate, in particular sodium salcaprozate (SNAC).

According to an embodiment, the composition comprises N-octanoyl-Phenylalanine, a peptide or a protein and a pH modifier.

According to an embodiment, the composition comprises N-octanoyl-Phenylalanine, a peptide or a protein and a pH modifier which is chosen from the group consisting of sodium carbonate, which formula is Na₂CO₃, phosphates, citrates, citric acid, tartarate and tartaric acid.

According to an embodiment, the composition comprises N-octanoyl-Phenylalanine, a peptide or a protein, a pH modifier and a protease inhibitor.

According to an embodiment, the composition comprises N-octanoyl-Phenylalanine, a peptide or a protein, a further permeation enhancer and a protease inhibitor.

According to an embodiment, the composition comprises N-octanoyl-Phenylalanine, a peptide or a protein, a lubricant and a protease inhibitor.

According to an embodiment, the composition comprises N-octanoyl-Phenylalanine, a peptide or a protein, a pH modifier, a further permeation enhancer and a protease inhibitor.

According to an embodiment, the composition comprises the AC-aa with an AC group comprising 8 carbon atoms, a peptide or a protein, and a lubricant which is magnesium stearate.

According to an embodiment, the composition comprises the AC-aa with an AC group comprising 8 carbon atoms, a peptide or a protein, and a lubricant which is glyceryl dibehenate.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein and a lubricant.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein and a lubricant which is magnesium stearate.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein and a lubricant which is glyceryl dibehenate.

According to an embodiment, the composition comprises the AC-aa with an AC group comprising 8 carbon atoms, a peptide or a protein and a pH modifier.

According to an embodiment, the composition comprises the AC-aa with an AC group comprising 8 carbon atoms, a peptide or a protein and a pH modifier which is sodium carbonate.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein and a pH modifier.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein and a pH modifier which is sodium carbonate.

According to an embodiment, the composition comprises the AC-aa with an AC group comprising 8 carbon atoms, a peptide or a protein, a lubricant and a pH modifier.

According to an embodiment, the composition comprises the AC-aa with an AC group comprising 8 carbon atoms, a peptide or a protein, a lubricant which is magnesium stearate and a pH modifier.

According to an embodiment, the composition comprises the AC-aa with an AC group comprising 8 carbon atoms, a peptide or a protein, a lubricant which is magnesium stearate and a pH modifier which is sodium carbonate.

According to an embodiment, the composition comprises the AC-aa with an AC group comprising 8 carbon atoms, a peptide or a protein, a lubricant which is glyceryl dibehenate and a pH modifier.

According to an embodiment, the composition comprises the AC-aa with an AC group comprising 8 carbon atoms, a peptide or a protein, and a lubricant which is glyceryl dibehenate and a pH modifier which is sodium carbonate.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein, a lubricant and a pH modifier.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein, a lubricant and a pH modifier which is sodium carbonate.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein, a lubricant which is magnesium stearate and a pH modifier.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein, a lubricant which is magnesium stearate and a pH modifier which is sodium carbonate.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein, a lubricant which is glyceryl dibehenate and a pH modifier.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein, a lubricant which is glyceryl dibehenate and a pH modifier which is sodium carbonate.

According to an embodiment, the composition comprises the AC-aa with an AC group comprising 8 carbon atoms, a peptide or a protein, a lubricant and a disintegrant.

According to an embodiment, the composition comprises the AC-aa with an AC group comprising 8 carbon atoms, a peptide or a protein, a lubricant which is magnesium stearate and a disintegrant.

According to an embodiment, the composition comprises the AC-aa with an AC group comprising 8 carbon atoms, a peptide or a protein, a lubricant which is glyceryl dibehenate and a disintegrant.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein, a lubricant and a disintegrant.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein, a lubricant which is magnesium stearate and a disintegrant.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein, a lubricant which is glyceryl dibehenate and a disintegrant.

According to an embodiment, the composition comprises the AC-aa with an AC group comprising 8 carbon atoms, a peptide or a protein, a lubricant, a pH modifier and a disintegrant.

According to an embodiment, the composition comprises the AC-aa with an AC group comprising 8 carbon atoms, a peptide or a protein, a lubricant which is magnesium stearate,a pH modifier and a disintegrant.

According to an embodiment, the composition comprises the AC-aa with an AC group comprising 8 carbon atoms, a peptide or a protein, a lubricant which is magnesium stearate, a pH modifier which is sodium carbonate and a disintegrant.

According to an embodiment, the composition comprises the AC-aa with an AC group comprising 8 carbon atoms, a peptide or a protein, a lubricant which is glyceryl dibehenate, a pH modifier and a disintegrant.

According to an embodiment, the composition comprises the AC-aa with an AC group comprising 8 carbon atoms, a peptide or a protein, and a lubricant which is glyceryl dibehenate, a pH modifier which is sodium carbonate and a disintegrant.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein, a lubricant, a pH modifier and a disintegrant.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein, a lubricant, a pH modifier which is sodium carbonate and a disintegrant.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein, a lubricant which is magnesium stearate, a pH modifier and a disintegrant.

According to an embodiment, the composition comprises AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein, a lubricant which is magnesium stearate, a pH modifier which is sodium carbonate and a disintegrant.

According to an embodiment, the composition comprises the AC-aa with an AC group comprising 8 carbon atoms and an aa chosen from aromatic amino acid selected from the group consisting of Phenylalanine (Phe), Phenylglycine (PGly), Tryptophane (Trp) and Tyrosine (Tyr), a peptide or a protein, a lubricant which is glyceryl dibehenate, a pH modifier and a disintegrant.

According to an embodiment, the composition comprises NaPheC8, a peptide or a protein, a lubricant which is glyceryl dibehenate, a pH modifier which is sodium carbonate and a disintegrant.

According to an embodiment, the composition comprises NaPheC8, a peptide or a protein, a lubricant, a pH modifier and a disintegrant.

According to an embodiment, the composition comprises NaPheC8, a peptide or a protein, a lubricant, a pH modifier which is sodium carbonate and a disintegrant.

According to an embodiment, the composition comprises NaPheC8, a peptide or a protein, a lubricant, a pH modifier, a disintegrant and a protease inhibitor.

According to an embodiment, the composition comprises NaPheC8, a peptide or a protein, a lubricant, a pH modifier which is sodium carbonate, a disintegrant and a protease inhibitor.

According to an embodiment, the composition comprises NaPheC8, a peptide or a protein, a lubricant which is magnesium stearate, a pH modifier and a disintegrant.

According to an embodiment, the composition comprises NaPheC8, a peptide or a protein, a lubricant which is magnesium stearate, a pH modifier which is sodium carbonate and a disintegrant.

According to an embodiment, the composition comprises NaPheC8, a peptide or a protein, a lubricant which is glyceryl dibehenate, a pH modifier and a disintegrant.

According to an embodiment, the composition comprises NaPheC8, a peptide or a protein, a lubricant which is glyceryl dibehenate, a pH modifier which is sodium carbonate and a disintegrant.

In an embodiment, the composition comprises an AC-aa, in particular according to Formula I, more particularly according to Formula la, and still more particularly an AC aa with AC being -CO(R1) and R1 being a C7 or C8 alkyl and a protein or a peptide.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, and a further permeation enhancer, in particular SNAC.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, and a protease inhibitor, in particular chosen from the group consisting of SBTI, BBI and KTI.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, and a protease inhibitor, in particular chosen from the group consisting of SBTI, BBI and KTI and their mixtures, and in particular SBTI, from 100 to 400 mg/g.

In an embodiment the composition comprises an AC-aa, in particular according to Formula I, more particularly according to Formula la, and still more particularly an AC aa with AC being -CO(R1) and R1 being a C7 or C8 alkyl, a protein or a peptide, and a protease inhibitor, in particular chosen from the group consisting of SBTI, BBI and KTI and their mixtures, and in particular SBTI, from 120 to 300 mg/g.

In an embodiment the composition comprises an AC-aa, in particular according to Formula I, more particularly according to Formula la, and still more particularly an AC aa with AC being -CO(R1) and R1 being a C7 or C8 alkyl, a protein or a peptide, and a protease inhibitor, in particular chosen from the group consisting of SBTI, BBI and KTI and their mixtures, and in particular SBTI, from 140 to 250 mg/g.

In an embodiment the composition comprises an AC-aa, in particular according to Formula I, more particularly according to Formula la, and still more particularly an AC aa with AC being -CO(R1) and R1 being a C7 or C8 alkyl, a protein or a peptide, and a protease inhibitor, in particular chosen from the group consisting of SBTI, BBI and KTI and their mixtures, and in particular SBTI, from 150 to 200 mg/g.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, and a chelator of divalent cation, in particular EDTA.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, and a pH modifier, in particular carbonate, more particularly Na2CO3.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, and a disintegrant, in particular croscarmellose.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a further permeation enhancer, in particular SNAC and a protease inhibitor, in particular chosen from the group consisting of SBTI, BBI and KTI.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a further permeation enhancer, in particular SNAC and a chelator of divalent cation, in particular EDTA.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a further permeation enhancer, in particular SNAC and a pH modifier, in particular carbonate, more particularly Na2CO3.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a further permeation enhancer, in particular SNAC, and a disintegrant, in particular croscarmellose.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a protease inhibitor, in particular chosen from the group consisting of SBTI, BBI and KTI, and a chelator of divalent cation, in particular EDTA.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a protease inhibitor, in particular chosen from the group consisting of SBTI, BBI and KTI, and a pH modifier, in particular carbonate, more particularly Na2CO3.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a protease inhibitor, in particular chosen from the group consisting of SBTI, BBI and KTI, and a disintegrant, in particular croscarmellose.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a further permeation enhancer, in particular SNAC, a protease inhibitor, in particular chosen from the group consisting of SBTI, BBI and KTI, and a chelator of divalent cation, in particular EDTA.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a further permeation enhancer, in particular SNAC, a protease inhibitor, in particular chosen from the group consisting of SBTI, BBI and KTI, and a pH modifier, in particular carbonate, more particularly Na2CO3.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a further permeation enhancer, in particular SNAC, a protease inhibitor, in particular chosen from the group consisting of SBTI, BBI and KTI, and a disintegrant, in particular croscarmellose.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a further permeation enhancer, in particular SNAC, a chelator of divalent cation, in particular EDTA, and a pH modifier, in particular carbonate, more particularly Na2CO3.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a further permeation enhancer, in particular SNAC, a chelator of divalent cation, in particular EDTA, and a disintegrant, in particular croscarmellose.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a protease inhibitor, in particular chosen from the group consisting of SBTI, BBI and KTI, a chelator of divalent cation, in particular EDTA, and a pH modifier, in particular carbonate, more particularly Na2CO3.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a protease inhibitor, in particular chosen from the group consisting of SBTI, BBI and KTI, a chelator of divalent cation, in particular EDTA, and a disintegrant, in particular croscarmellose.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a protease inhibitor, in particular chosen from the group consisting of SBTI, BBI and KTI, a pH modifier, in particular carbonate, more particularly Na2CO3, and a disintegrant, in particular croscarmellose.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a further permeation enhancer, in particular SNAC, a protease inhibitor, in particular chosen from the group consisting of SBTI, BBI and KTI, a chelator of divalent cation, in particular EDTA, and a pH modifier, in particular carbonate, more particularly Na2CO3.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a further permeation enhancer, in particular SNAC, a protease inhibitor, in particular chosen from the group consisting of SBTI, BBI and KTI, a chelator of divalent cation, in particular EDTA, and a disintegrant, in particular croscarmellose.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a further permeation enhancer, in particular SNAC, a protease inhibitor, in particular chosen from the group consisting of SBTI, BBI and KTI, a pH modifier, in particular carbonate, more particularly Na2CO3, and a disintegrant, in particular croscarmellose.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a further permeation enhancer, in particular SNAC, a chelator of divalent cation, in particular EDTA, a pH modifier, in particular carbonate, more particularly Na2CO3, and a disintegrant, in particular croscarmellose.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a protease inhibitor, in particular chosen from the group consisting of SBTI, BBI and KTI, a chelator of divalent cation, in particular EDTA, a pH modifier, in particular carbonate, more particularly Na2CO3, and a disintegrant, in particular croscarmellose.

In an embodiment, the composition comprises an AC-aa, in particular according to Formula I, more particularly according to Formula la, and still more particularly an AC aa with AC being -CO(R1) and R1 being a C7 or C8 alkyl, a protein or a peptide, a further permeation enhancer, in particular SNAC, a protease inhibitor, in particular chosen from the group consisting of SBTI, BBI and KTI, a chelator of divalent cation, in particular EDTA, a pH modifier, in particular carbonate, more particularly Na2CO3, and a disintegrant, in particular croscarmellose.

### EXAMPLES

The AC-aa may be obtained by acylation of amino-acids, which may be readily performed using acylation agents known in the art that react with e.g. the free alpha-amino group. The amino acid may be attached to the acid via an N-acylation, i.e. resulting in an amide bond. AC-aa's of the invention may be prepared using the method described in Leone-Bay et al (1995): "N-acylated alpha-amino acids as novel oral delivery agents for proteins", Journal of Medicinal Chemistry, 38(21), 4263-4269.

### Part A - Preparation of compositions

### A.1. Solid compositions

### Process A-1

The solid compositions were prepared by mixing together the AC-aa and other excipients with the peptide or a protein in appropriate proportions using a mortar and pestle until a sufficient homogeneity was reached. The resulting blend was then introduced manually in Enteric VCaps, size 00 (Capsugel).

Capsules were then sealed using the following protocol: 10µL of a 50/50 vol/vol mixture of ethanol and water were introduced between the lower part and the upper lid of the capsule using a syringe. The sealing solution was then dried under hot air (45°C) for one minute, leading to an impermeable seal between both parts of the capsule.

### Example A.1.1 : Preparation a NaPheC8 and semaglutide solid composition.

The composition A.1.1 is prepared according to process A-1 using 15 mg of semaglutide and 395 mg of NaPheC8 per enteric cap.

### Process A-2

The solid compositions were prepared by first mixing together the AC-aa with the peptide or a protein in appropriate proportions, as well as other excipients in aqueous solution. Total solute concentration is in the range 20-500mg/g of solution. This solution is then freeze-dried to yield a homogeneous solid lyophilisate. A mortar and pestle is then used to crush this solid into a powder suitable for further processing. The resulting blend was then introduced manually in Enteric VCaps, size 00 (Capsugel).

Capsules were then sealed using the following protocol: 10µL of a 50/50 vol/vol mixture of ethanol and water were introduced between the lower part and the upper lid of the capsule using a syringe. The sealing solution was then dried under hot air (45°C) for one minute, leading to an impermeable seal between both parts of the capsule.

### Example A.2.1: Preparation a NaPheC8 and semaglutide solid composition.

Composition A.2.1 is prepared according to process A.2 using 14mg of semaglutide, 400mg of NaPheC8, 43mg of sodium carbonate and 20mg of SBTI.

### Part B - Physico-chemistry

### Example B1: Determination of solubilization kinetics of solid NaPheC6 and NaPheC8

To evaluate solubilization kinetics of NaPheC6 and NaPheC8 in similar conditions, compacts are prepared in the following manner:

In a manual tablet press (LFA TDP 0 Desktop tablet press) equipped with cylindrical 8 mm diameter punches, 200 ± 10mg of permeation enhancer is filled into the die cavity. Compression into cylindrical compacts of 4 ± 0.5 mm height yields compacts with apparent volumetric mass of 1 ± 0.2 g/mL.

These compacts are put into a capsule sinker, then dissolved using an USP < 711 >-compliant apparatus (type 2-Paddle apparatus). Dissolution is done into 700 mL of 200 mM phosphate buffer, pH 6.8. Temperature is set at 37°C and agitation at 75 rpm.

Sampling of the dissolution medium followed by HPLC dosage yields the following dissolution profiles for the compounds NaPheC6 and NaPheC8, respectively:

| Time (seconds) | NaPheC6 dissolved (%) | NaPheC8 dissolved (%) |
|---|---|---|
| 20 | 47 | 15 |
| 40 | 100 | 37 |
| 60 | 100 | 57 |
| 80 | 100 | 89 |
| 100 | 100 | 99 |

One can see that compounds of the invention are readily soluble and lead to very rapid dissolution in these testing conditions. This can be advantageous to reach an immediate release upon disintegration and dissolution of an oral dosage form.

### Part C - Pharmacokinetics

### Example C1 : Pharmacokinetic studies in Beagle dogs

Pharmacokinetic (PK) studies in Beagle dogs were conducted to estimate the exposure and the bioavailability of semaglutide after oral administration of the composition A.1.1 comprising semaglutide and NaPheC8 described in example A.1.1 and after intravenous (iv) administration of semaglutide. As a reference, Rybeslus^{®} (oral semaglutide from Novo Nordisk) was orally administered in a separate study.

For the oral administration, 10 Beagle dogs weighing approximately 10 kg, were fasted overnight before the start of the experiment and from 0 to 6h after dosing. The capsule containing the composition were placed at room temperature 15 minutes before administration, and not more than 30 minutes. Each dog received orally a capsule containing the composition A.1.1 comprising semaglutide and NaPheC8 described in example A.1.1. Immediately after oral administration, 5mL of water was administered using a syringe to facilitate swallowing.

For the intravenous (iv) administration, a solution of semaglutide (10 µmol/L) was injected through a catheter, placed in the cephalic vein, to 10 Beagle dogs.

Blood was sampled at predefined time up to 72h for oral administration and up to 192h for the iv administartion. Each blood sampling time point was collected in K₂EDTA tubes containing a cocktail of protease inhibitors stored less than 45min on ice before centrifugation. The centrifugation was performed as follows: 10 min at 1300 G at +4°C. Plasma was collected in two cryotubes with minimum 100µL in each one. Cryotubes are stored at -80°C until analysis

Plasma concentrations of semaglutide were determined using a LC-MS/MS analysis after protein precipitation extraction.

Semaglutide plasma concentration data corrected for actual dose and body weight were subjected to non-compartmental PK analysis using Phoenix WinNonlin V8.2 software (Pharsight, Mountain View, Calif. 94041, USA). For each individual dog, the following parameters were estimated: maximum plasma concentration (Cmax), time for maximal concentration (tmax) and area under the curve from 0 to infinity (AUCinf). Bioavailability (F) was calculated as the fraction absorbed (in %) based on the ratio of dose-normalised AUCinf after oral and iv administration ((AUCinf-oral/dose)/(AUCinfiv/dose)). Summary statistics of PK parameters were presented as median and arithmetic mean with corresponding standard deviation (SD). In the case of high intersubject variability with a small sample size, the median appears to be a better estimator of the PK parameters than the arithmetic mean (i.e. no influence of extreme values).

### Results

Individual median and mean (SD) calculated PK parameters following oral administration of the composition A.1.1 comprising semaglutide and NaPheC8 described in example A.1.1 are reported in the following table:

| Pharmacokinetic parameters for semaglutide following oral dosing of the combination of semaglutide and NaPheC8 to 10 male Beagle dogs | | | | |
|---|---|---|---|---|
| Dog no | tmax (h) | Cmax (ng/mL) | AUCinf (h* ng/mL) | F (%) |
| 1 | 0.75 | 29.5 | 1785 | 0.25 |
| 2 | 8 | 156.0 | 12176 | 1.31 |
| 3 | 2 | 269.3 | 18682 | 2.75 |
| 4 | 0.75 | 119.7 | 7037 | 0.67 |
| 5 | 1.5 | 6.4 | 476 | 0.05 |
| 6 | 1.5 | 23.2 | 1245 | 0.18 |
| 7 | 0.5 | 14.5 | 996 | 0.11 |
| 8 | 0.5 | 3.2 | 328 | 0.04 |
| 9 | 0.75 | 14.9 | 805 | 0.07 |
| 10 | 1 | 20.5 | 1358 | 0.15 |
| Median | 1.000 | 21.9 | 1302 | 0.17 |
| Mean | 1.725 | 65.7 | 4489 | 0.56 |
| SD | 2.259 | 88.4 | 6270 | 0.87 |

Individual, median and mean (SD) calculated PK parameters following oral administration of Rybelsus^{®} are reported in the following table:

| Pharmacokinetic parameters for semaglutide following oral dosing of Rybelsus^{®} to 10 male Beagle dogs | | | | |
|---|---|---|---|---|
| Dog no | tmax (h) | Cmax (ng/mL) | AUC0-5 (h^{∗}ng/mL) | F (%) |
| 1 | 1.0 | 64.7 | 261 | 0.34 |
| 2 | 3.0 | 3070.0 | 11299 | 16.56 |
| 3 | 2.0 | 152.0 | 628 | 0.89 |
| 4 | 3.0 | 8.3 | 34 | 0.05 |
| 5 | 3.0 | 8.0 | 36 | 0.05 |
| 6 | 2.0 | 18.4 | 76 | 0.11 |
| 7 | 1.0 | 32.7 | 145 | 0.19 |
| 8 | 0.75 | 51.1 | 177 | 0.24 |
| 9 | 1.0 | 74.1 | 296 | 0.39 |
| 10 | 0.75 | 24.5 | 91 | 0.12 |
| Median | 1.500 | 41.9 | 161 | 0.21 |
| Mean | 1.750 | 350.4 | 1305 | 1.89 |
| SD | 0.972 | 273.0 | 3516 | 5.16 |

The results showed that absorption was observed with all individual dogs following oral administration of composition A.1.1 comprising semaglutide and NaPheC8 described in Example A.1.1. The bioavailability of semaglutide ranged from 0.04% to 2.75% for composition A.1.1 comprising semaglutide and NaPheC8 described in Example A.1 and from 0.05% to 16.56% for Rybelsus^{®} described in Example C1. Based on median and SD values, composition A.1.1 comprising semaglutide and NaPheC8 described in Example A.1 showed a comparable bioavailability (median F(%): 0.17% vs. 0.21%) with a lower intersubject variability (SD: 0.87% vs. 5.16%) compared to Rybelsus^{®} .

### Example C2: Pharmacokinetic studies in Beagle dogs

Pharmacokinetic (PK) studies in Beagle dogs were conducted to estimate the exposure and the bioavailability of semaglutide after oral administration of the composition A.2.1 comprising semaglutide, NaPheC8 and SBTI described in example A.2

A protocol similar to that described in Example C1 was used. However, blood was only sampled up to 5h.

Semaglutide plasma concentration data were subjected to non-compartmental PK analysis using Phoenix WinNonlin V8.3 software. (Pharsight, Mountain View, Calif. 94041, USA). For each individual dog, the following parameters were estimated: maximum plasma concentration (Cmax), time for maximal concentration (tmax) and area under the curve from 0 to 5h (AUC0-5h). Since blood sampling was stopped after 5h, extrapolation of the AUC to infinity was not reliable. Therefore, the bioavailability (F) of the composition A.2.1 was estimated as the maximum cumulative fraction input obtained from plasma PK deconvolution. The unit impulse response (UIR) function used for deconvolution was derived from the IV injection described in Example C1. Summary statistics of PK parameters were presented as median and arithmetic mean with corresponding standard deviation (SD).

### Results

Individual, median and mean (SD) calculated PK parameters following oral administration of the composition A.2.1 comprising semaglutide, NaPheC8 and SBTI described in Example C2 are reported in the following table:

| Pharmacokinetic parameters for semaglutide following oral dosing of the combination of semaglutide, NaPheC8 and SBTI to 10 male Beagle dogs | | | | |
|---|---|---|---|---|
| Dog no | tmax (h) | Cmax (ng/mL) | AUC0-5 (h^{∗} ng/mL) | F (%) |
| 1 | 1.5 | 52.3 | 190.6 | 0.31 |
| 2 | 2.0 | 208.0 | 859.1 | 1.33 |
| 3 | 1.0 | 63.5 | 261.7 | 0.41 |
| 4 | 2.0 | 40.3 | 164.8 | 0.26 |
| 5 | 2.0 | 91.0 | 381.7 | 0.67 |
| 6 | 1.0 | 12.4 | 47.3 | 0.08 |
| 7 | 0.75 | 240.0 | 984.0 | 1.57 |
| 8 | 0.75 | 68.1 | 263.9 | 0.42 |
| 9 | 2.0 | 158.0 | 632.2 | 1.09 |
| 10 | 1.5 | 30.5 | 108.2 | 0.18 |
| Median | 1.500 | 65.8 | 263 | 0.41 |
| Mean | 1.450 | 96.4 | 389 | 0.60 |
| SD | 0.537 | 78.4 | 32 | 0.50 |

The results showed that absorption was observed with all individual dogs following oral administration of composition A.2.1 comprising semaglutide, NaPheC8 and SBTI described in Example A.2. The bioavailability of semaglutide ranged from 0.08% to 1.57% for composition A.2.1 comprising semaglutide, NaPheC8 and SBTI described in Example A.2.1, and from 0.05% to 16.56% for Rybelsus^{®}, as shown in Example C1. Based on median and SD values, composition A.2.1 comprising semaglutide, NaPheC8 and SBTI described in Example A.2.1 showed a greater bioavailability (median F(%): 0.41% vs. 0.21%) with a lower intersubject variability compared to Rybelsus^{®} (SD: 0.50% vs. 5.16%) compared to Rybelsus^{®} described in Example C1.

### Part D - In vitro biology

### Example D1: In vitro enzymatic degradation

An *in vitro* enzymatic degradation study was conducted in order to evaluate the protective effect of SBTI, NaPheC8 and combinations thereof regarding enzymatic degradation. Porcine pancreatine dissolved in Fasted State Simulated Intestinal Fluid (FasSIF, pH = 6.5 +/- 0.2) was used as a model of intestinal enzymatic degradation.

Formulations containing semaglutide (50 mg/mL), semaglutide + NaPheC8, semaglutide + SBTI and semaglutide + SBTI + NaPheC8 at relevant API/NaPheC8/SBTI ratio. Pancreatin (5 U/mL) was added to the formulation at T = 0 min to initiate the degradation process by gentle mixing. All samples were incubated at 37°C under 100 rpm agitation. Samples were withdrawn at the time intervals 0 min, 5 min, 15 min, 30 min and 45 min and enzyme activity was quenched to allow quantitative determination of intact peptide remaining in the solution.

Samples were then analyzed by RP-HPLC with UV detection (lower quantification limit = 0.7 µg/mL, corresponding to 1.4% of initial semaglutide concentration).

### Results

Percentage of intact semaglutide (compared to t=0) are reported in the table below:

| **Degradation kinetics of semaglutide in 5 U/ mL pancreatin (% remaining intact peptide, N=1)** *(N.D. = not detected)* | | | | |
|---|---|---|---|---|
| Time (min) | **Semaglutide** | **Sem aglutide + NaPheC8** | **Semaglutide + SBTI** | **Sem aglutide + SBTI + NaPheC8** |
| 0 | 100.0 | 100.0 | 100.0 | 100.0 |
| 5 | 9.1 | 49.1 | 90.3 | > 95% |
| 15 | N.D. | 9.1 | 75.7 | > 95% |
| 30 | N.D. | N.D. | 61.0 | > 95% |
| 45 | N.D. | N.D. | 43.6 | > 95% |

Semaglutide is rapidly degraded in pancreatin as only 9.1% intact peptide remained after 5 min incubation, and intact peptide was undetectable after 15 min. NaPheC8 significantly slowed down semaglutide degradation kinetics (49.1% intact peptide present after 5 min, 9.1% after 15 min).

SBTI protects semaglutide from pancreatin enzymatic degradation as 43.6% of intact peptide are still present after 45 min incubation. The co-formulation of NaPheC8 with SBTI significantly impacts enzyme degratation as 99.3% of intact semaglutide are observed after 45 min, showing the interest of the co-formulation of NaPheC8 and SBTI for the protection of peptides against enzyme degradation.

## Claims

1. Solid composition comprising a peptide or a protein and an acylated aminoacid AC-aa, or a salt thereof, **characterized in that**:
- the acyl group -AC is (C(O)R1) wherein R1 is an alkyl group comprising from 3 to 8 carbon atoms,
- said composition comprises at least 300 mg/g of AC-aa relative to the total weight of the composition.

2. Composition according to Claim 1, **characterized in that** the composition is an oral composition.

3. Solid composition according to any one of the preceding Claims, **characterized in that** AC-aa is chosen amongst the AC-aa wherein -aa is an amino acid and has the general Formula I :
R3OOC-CH(R4)-(X)n-N(R2)-C(=O)R1 Formula I
wherein :
- X is
o -CH(R4)-(CH2)n- with n being 0, 1 or 2,
or
o a divalent 6 carbon aromatic ring (benzene), such benzene ring bearing R3OOC- and -N(R2)-C(=O)R1 in 1,2, 1,3 or 1,4 positions, in particular the benzene group does bear only R3OOC- and -N(R2)-C(= O)R1 substituents,
- -R1 is an alkyl group comprising from 3 to 8 carbon atoms,
- -R2 is
o an hydrogen atom (-H) or a methyl group -(CH₃) and -R4 is an amino acide side chain or
o R2 and R4 form together a -(CH₂)ₘ- group, with m being an integer chosen from 3 and 4 when n = 0, from 2 and 3 when n = 1, and 2 when n = 2
- -R3 is -H, -Na or -K.

4. Solid composition according to any one of the preceding Claims, **characterized in that** AC-aa is chosen amongst the AC-aa wherein -aa is an alpha amino acid and AC-aa has the general Formula Ia : 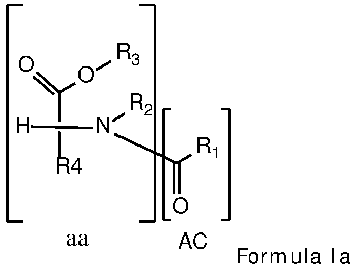 wherein :
- -R1 is an alkyl group comprising from 3 to 8 carbon atoms,
- -R2 is an hydrogen atom (-H), a methyl group -(CH₃) or is covalently attached to R4 via a -(CH₂) - group,
- -R3 is -H, -Na or -K,
- -R4 is an amino acid side chain or is covalently attached to -R2 via a -(CH₂)₂₋ or
-(CH₂)₃- group.

5. Composition according to Claim 3, **characterized in that** R1 is a linear alkyl group.

6. Solid composition according to any of the preceding Claims, **characterized in that** the -aa is issued from a non cationic amino acid, a non polar hydrophobic amino acid, a polar non charged amino acid or a polar acidic amino acid.

7. Solid composition according to any of the preceding Claims, **characterized in that** the AC-aa is selected from the group consisting of N-octanoyl-Phenylalanine, N-octanoyl-Phenylglycine, N-octanoyl-Tryptophane and N-octanoyl-Tyrosine.

8. Solid composition according to any of the preceding Claims, **characterized in that** the AC-aa has a critical micellar concentration in solution in water in biorelevant's FASSIF buffer at pH 6.5 and 25°C, also called CMC, of at least 1 mM..

9. Solid composition according to any of the preceding Claims, **characterized in that** it comprises a further permeation enhancer.

10. Solid composition according to any of the preceding Claims, **characterized in that** the the composition comprises from 0.5 to 20 wt% of a peptide or protein.

11. Solid composition according to any of the preceding Claims, **characterized in that** the peptide or protein is chosen from the group consisting of GLP-1 RA, GLP-2 RA, insulin and insulin analogs, amylin RA, GIP RA, PYY RA, dual agonists GIP/glucagon, dual agonists GLP-1/glucagon, ParaThyroid Hormones (PTH) and PTH analogs, InterLeukines (IL) and IL analogs, Growth Hormones (GH) and GH analogs, Insulin Growth Factors (IGF) and IGF analogs, Interferons (IFN) and IFN analogs.

12. Solid composition according to any of the preceding Claims, **characterized in that** it further comprises a protease inhibitor different from AC-aa.

13. Solid composition according to any of the preceding Claims, **characterized in that** it comprises a lubricant.

14. Solid composition according to any of the preceding Claims, **characterized in that** it comprises a pH modifier.

15. Solid composition according to any of the preceding Claims, for use as a medicament.

16. Solid composition according to Claim 15, for preventing or treating obesity, Diabetes Type 1, Diabetes Type 2, NASH, thrombocytopaenia, Short Bowel Syndrom (SBS), adult GH deficiency, GH disorders, Short stature syndrome, Turner's syndrome, Achondroplasia, Prader-Willi syndrome, Short stature in children, osteoporosis and hypoparathyroidism, Multiple sclerosis, Bone disorders.

17. Unitary solid dosage comprising a peptide or a protein and at least 300 mg/g of one acylated aminoacid, also called AC-aa, or a salt thereof, wherein the acyl group -AC is (C(O)R1) wherein R1 is an alkyl group comprising from 3 to 8 carbon atoms, in particular 3 to 7 carbon atoms, wherein the unitary solid dosage comprises at least 50 mg of AC-aa.
